(19) Europäisches Patentamt
European Patent Office
Office européen des brevets

(11) **EP 3 115 916 B1**

(12) **EUROPEAN PATENT SPECIFICATION**

(45) Date of publication and mention
of the grant of the patent:
**23.04.2025 Bulletin 2025/17**

(51) International Patent Classification (IPC):
**A61B 5/00** *(2006.01)* **G10H 1/40** *(2006.01)*
**A61B 5/12** *(2006.01)*

(21) Application number: **15306104.9**

(22) Date of filing: **06.07.2015**

(52) Cooperative Patent Classification (CPC):
**A61B 5/12; A61B 5/4005; A61B 5/725; G10H 1/40;
G16H 50/20;** G10H 2210/076; G10H 2210/091;
G10H 2240/325

(54) **METHOD AND APPARATUS FOR THE SYNCHRONIZATION OF DATA SEQUENCES INCLUDING FILTERING**

VERFAHREN UND VORRICHTUNG ZUR SYNCHRONISIERUNG VON DATENSEQUENZEN EINSCHLIESSLICH FILTRIERUNG

PROCÉDÉ ET APPAREIL POUR LA SYNCHRONISATION DE SÉQUENCES DE DONNÉES COMPRENANT LE FILTRAGE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(43) Date of publication of application:
**11.01.2017 Bulletin 2017/02**

(73) Proprietor: **UNIVERSITE DE MONTPELLIER**
**34090 Montpellier (FR)**

(72) Inventors:
• **DALLA BELLA, Simone**
**34830 Clapiers (FR)**
• **KOTZ, Sonja**
**04155 Leipzig (DE)**

(74) Representative: **Gevers & Orès**
**Immeuble le Palatin 2**
**3 Cours du Triangle**
**CS 80165**
**92939 Paris La Défense Cedex (FR)**

(56) References cited:
• **BROCHARD R ET AL: "Evidence of beat perception via purely tactile stimulation", BRAIN RESEARCH, ELSEVIER, AMSTERDAM, NL, vol. 1223, 5 August 2008 (2008-08-05), pages 59 - 64, XP023176669, ISSN: 0006-8993, [retrieved on 20080528], DOI: 10.1016/ J.BRAINRES.2008.05.050**

• **KRAUSE V ET AL: "Functional network interactions during sensorimotor synchronization in musicians and non-musicians", NEUROIMAGE, ACADEMIC PRESS, ORLANDO, FL, US, vol. 52, no. 1, 1 August 2010 (2010-08-01), pages 245 - 251, XP027093285, ISSN: 1053-8119, [retrieved on 20100617]**

• **CHARLES-ETIENNE BENOIT ET AL: "Development and validation of a geriatric depression screening scale: a preliminary report", FRONTIERS IN HUMAN NEUROSCIENCE, vol. 17, no. 10 Pt 2, 7 July 2014 (2014-07-07), pages 37, XP055243359, DOI: 10.1016/0022-3956(82)90033-4**

• **BRUNO H REPP: "Multiple temporal references in sensorimotor synchronization with metrical auditory sequences", PSYCHOLOGICAL RESEARCH PSYCHOLOGISCHE FORSCHUNG ; AN INTERNATIONAL JOURNAL OF PERCEPTION, ATTENTION, MEMORY, AND ACTION, SPRINGER, BERLIN, DE, vol. 72, no. 1, 25 May 2006 (2006-05-25), pages 79 - 98, XP019563648, ISSN: 1430-2772**

• **JAKUB SOWINSKI ET AL: "Poor synchronization to the beat may result from deficient auditory-motor mapping", NEUROPSYCHOLOGIA, vol. 51, no. 10, 1 August 2013 (2013-08-01), GB, pages 1952 - 1963, XP055243095, ISSN: 0028-3932, DOI: 10.1016/j.neuropsychologia.2013.06.027**

EP 3 115 916 B1

**Description**

The present invention concerns a method and a device for the synchronization of data sequences including filtering. This method is particularly of interest in a device to test the rhythmic skills of a person.

There is an increasing interest in research and in clinical fields in the measurement of individuals' rhythmic skills. The majority of individuals, musicians and non-musicians alike, are capable of perceiving the rhythm of sound stimuli (e.g., music or speech). In addition, they can easily produce regular motor rhythms, and synchronize their movements to the beat of a sound signal, such as music. However, rhythmic skills can be disrupted in individuals with brain lesions, neurodegenerative disorders (e.g., Parkinson's disease), and in developmental disorders (e.g., dyslexia and ADHD). In particular the skill of synchronizing movement to the beat of an external rhythmic stimulus seems critically related to important cognitive functions such as working memory, executive functions (e.g., inhibition) and attention, some of which are deficient in the aforementioned populations. Thus, obtaining a thorough diagnosis of rhythm and in particular synchronization abilities is critical to devise dedicated intervention and remediation strategies, with the goal of improving cognitive abilities in impaired populations. The synchronization of movement to the beat of a regular sound stimulus (e.g., a metronome or music) is used to test individuals' rhythmic skills in a variety of populations. The most common paradigm used in the laboratory is based on finger or hand tapping on a sensitive surface (e.g., sensor of a MIDI percussion instrument, mouse or keyboard key on a computer, or tablet touchscreen). The dedicated tapping devices are linked to a sound-generation device capable of delivering highquality sound stimulation, such as a sequence of isochronous sounds or a musical excerpt. The devices used in tapping studies are capable of storing the timing of the finger/hand taps with a 1-ms precision relative to the timestamps of the presented sounds or musical beats. The stored timing information constitutes the raw data. This raw data needs to be synchronized to the time stamps of the pacing stimulus (e.g., times of individual sounds or of musical beats). For this reason, raw data is called synchronization data. The respective times of the sounds and of the taps are stored on the same time scale and thus are used to measure synchronization accuracy and variability also called consistency.

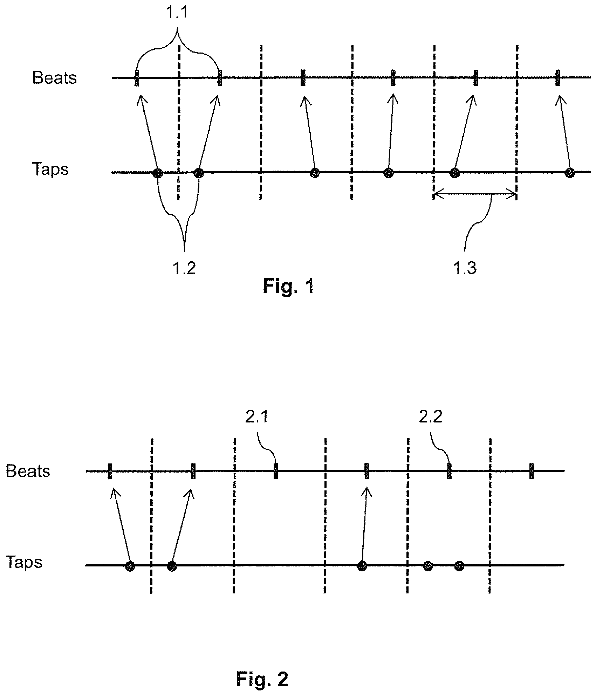

In order to obtain a very accurate and reliable measurement of a participant's rhythmic skills, raw data (tap times and sound/musical beat times) have to be submitted to thorough analyses. The vast majority of studies with the tapping paradigm tested individuals with excellent rhythmic skills who would perform the task quite proficiently. For these individuals, a typical performance implies a good 1-to-1 correspondence between the sounds and the taps, as illustrated in **figure 1.** In this case there is only 1 tap occurring in the interval around the beat stimuli.

**Figure 1** illustrates an example of synchronization data produced by a subject exhibiting excellent rhythmic skills. On this figure the first row illustrates the time line of the beats reference sound **1.1** occurring regularly. The second row illustrates the time line of the recorded taps **1.2** produced by the subject trying to synchronize to the reference beats. It happens that the subject is able to follow the reference beats quite well and in particular that each tap is related in a one-to-one relationship to a reference beat. Namely, if we split the time in intervals **1.3** centred on the reference beats, a tap belongs to only one interval and each interval provides a tap.

This data typically requires little filtering, and the raw data can be used as such to calculate the main measures of synchronization accuracy (e.g., the mean asynchrony between the time of the stimuli and the time of the pacing stimuli) and variability. However, analyzing synchronization is a much more challenging task when testing the performance of individuals with rhythm and movement disorders (e.g., patients with Parkinson's disease) or children, because of the variability of their performance. An example of a performance is provided in **figure 2.**

**Figure 2** illustrates another example of synchronization data produced by a subject exhibiting poor rhythmic skills. It may be noted that the one-to-one relationship between the beat references and the taps does no longer occur. For example, no tap may be identified corresponding to beat reference **2.1,** while two tap candidates may be associated to beat reference **2.2.**

In this case, where there is not a systematic one-to-one correspondence between the taps and the beat references, the data have to be adequately filtered. Lack of appropriate data filtering (i.e., removal of tap timing data or of inter-tap intervals) can lead to erroneous measurement of rhythmic skills, such as an underestimation of rhythmic skills. Filtering methods are even more critical with synchronization to a complex sound stimulus such as music, where synchronization can occur at different metrical levels (e.g., half, quarter or eighth note).

Brochard et al, 2008. Evidence of beat perception via purely tactile stimulation. Brain Research 1223, 59-64. Studied the perception of meter felt with tactile sensory inputs.

The present invention has been devised to address one or more of the foregoing concerns. It comprises new systematic procedures for filtering synchronization data (raw data: tap times and sound/musical beat reference times) in order to obtain accurate and reliable measures of synchronization accuracy and variability (or consistency). The procedures detailed below are used to provide reliable synchronization data that can be used to compute measures of synchronization accuracy and variability based on linear statistics or circular statistics. These procedures are particularly appropriate for analyzing synchronization performance in individuals with rhythm disorders.

The invention is defined in claim 1. According to a first aspect of the disclosure there is provided a method for the synchronization of data sequences, the method comprising by a computing device:

- obtaining a sequence of reference beat times;
- obtaining a sequence of recorded tap times by a subject;
- computing scores reflecting some rhythmic skills of the subject, said scores comprising at least the synchronization accuracy and the synchronization variability of said sequence of recorded tap times regarding said sequence of reference beat times;

characterized in that the method further comprises a filtering step prior to computing scores, the filtering step comprising:

- rejecting artefacts in the sequence of recorded tap times, artefacts being defined as a tap for which the inter-tap-interval between the actual tap and the previous one is smaller than a given threshold;
- rejecting outliers in the sequence of recorded tap times, outliers being defined as a tap for which the inter-tap-interval between the actual tap and the previous one is outside a given range; and
- selecting continuous sequences of taps in the sequence of recorded tap times; continuous sequence of taps being defined as a sequence of successive taps without any outliers and having a given minimum length.

In the invention, the given range is the range between a low threshold and a high threshold given by:

- low threshold = Q1 - 3.IQR; and
- high threshold = Q3 + 3.IQR;
  wherein Q1 is the first quartile, Q3 is the third quartile and IQR is the interquartile range of the inter-tap-interval sequence.

In an embodiment, the filtering step further comprises:

- selecting regular sequences of taps in the sequence of recorded tap times among continuous sequences; a regular sequence being a sequence of taps with a strict one-to-one relationship with the reference beats.

In an example not being part of the invention, the sequence of reference beat times corresponding to a musical excerpt with several metrical level, the filtering step further comprises:

- computing a resultant vector $\vec{R}$ according to circular statistics for each possible inter-beat-interval according to each possible metrical level in the music;
- determining the metrical level chosen by the subject as the inter-beat-interval corresponding to the maximum length of $\vec{R}$; and
- filtering the sequence of reference beat times to only take into account beats corresponding to the chosen metrical level.

In an embodiment, the step of computing scores comprises computing the synchronization accuracy and the synchronization variability of said sequence of recorded tap times regarding said sequence of reference beat times according to linear statistics.
In an example not being part of the invention, the step of computing scores comprises computing the synchronization accuracy and the synchronization variability of said sequence of recorded tap times regarding said sequence of reference beat times according to circular statistics.
According to a another aspect of the disclosure there is provided a device for the synchronization of data sequences, the device comprising:

- means for obtaining a sequence of reference beat times;
- means for obtaining a sequence of recorded tap times by a subject;
- means for computing scores reflecting some rhythmic skills of the subject, said scores comprising at least the synchronization accuracy and the synchronization variability of said sequence of recorded tap times regarding said sequence of reference beat times;
  characterized in that the device further comprises a filtering module comprising:

  - means for rejecting artefacts in the sequence of recorded tap times, artefacts being defined as a tap for which the inter-tap-interval between the actual tap and the previous one is smaller than a given threshold;
  - means for rejecting outliers in the sequence of recorded tap times, outliers being defined as a tap for which the inter-tap-interval between the actual tap and the previous one is outside a given range; and

- means for selecting continuous sequences of taps in the sequence of recorded tap times; continuous sequence of taps being defined as a sequence of successive taps without any outliers and having a given minimum length.

According to another aspect of the invention there is provided a computer program product for a programmable apparatus, the computer program product comprising a sequence of instructions for implementing a method according to the invention, when loaded into and executed by the programmable apparatus.

According to another aspect of the invention there is provided a computer-readable storage medium storing instructions of a computer program for implementing a method according to the invention.

At least parts of the methods according to the invention may be computer implemented. Accordingly, the present invention may take the form of an entirely hardware embodiment, an entirely software embodiment (including firmware, resident software, micro-code, etc.) or an embodiment combining software and hardware aspects that may all generally be referred to herein as a "circuit", "module" or "system". Furthermore, the present invention may take the form of a computer program product embodied in any tangible medium of expression having computer usable program code embodied in the medium.

Since the present invention can be implemented in software, the present invention can be embodied as computer readable code for provision to a programmable apparatus on any suitable carrier medium. A tangible carrier medium may comprise a storage medium such as a floppy disk, a CD-ROM, a hard disk drive, a magnetic tape device or a solid-state memory device and the like. A transient carrier medium may include a signal such as an electrical signal, an electronic signal, an optical signal, an acoustic signal, a magnetic signal or an electromagnetic signal, e.g. a microwave or RF signal.

Examples will now be described, by way of example only, and with reference to the following drawings in which:

figure 1 illustrates an example of synchronization data produced by a subject exhibiting excellent rhythmic skills;
figure 2 illustrates another example of synchronization data produced by a subject exhibiting poor rhythmic skills;
figure 3 illustrates the general process of synchronization
figure 4 illustrates the filtering process in the invention;
figure 5 illustrates how synchronization data are represented on a polar scale for the computation of circular statistics;
figure 6 is a schematic block diagram of a computing device for implementation of one or more examples.

The Battery for the Assessment of Auditory Sensorimotor and Timing Abilities (BAASTA) is a new tool for assessing perceptual and sensorimotor abilities in the general population. The broad set of tasks covers a range of abilities in interval timing, beat-based timing, and beat perception, as well as spontaneous motor behaviour (tapping) and sensorimotor synchronization tasks.

The set of perceptual tasks was devised to assess participants' ability to discriminate durations and to detect temporal deviations in rhythmical sequences. It comprises:

• a duration discrimination task where the subject is asked to discriminate between a 600 ms sound signal and a longer one;
• an anisochrony detection task with tones where the subject is asked whether sequences of tones contain or not an irregularity (i.e., an anisochrony);
• an anisochrony detection task with musical sequences where the subject is asked whether the beat of short musical excerpts contains or not an irregularity (i.e., an anisochrony) ; and
• a beat alignment task where the subject is asked if an isochronous sound signal superimposed to musical excerpts is aligned to the beat of music or not.

Sensorimotor tasks serve to assess participants' production abilities. It comprises:

• an unpaced tapping task where the subject is asked to tap with her/his index finger or hand at a regular pace without any stimuli;
• a paced tapping task with sequences of tones and with musical stimuli where the subject is asked to produce a sequence of taps synchronized to a reference sound signal. The reference sound signal may be the onset of the sequence of tones or the beat of a musical sequence;
• a synchronization-continuation task where the subject is asked to firstly synchronize tapping to a reference sound signal and secondly to continue tapping regularly after the disappearance of the reference sound signal;
• an adaptive tapping task identical to the previous one with the difference that the reference sound signal may be subject to a tempo change in its last part.

A comprehensive description of BAASTA may be found in the article: Benoit, C-E., Dalla Bella, S., Farrugia, N.,

Obrig, H., Mainka, S., & Kotz, S.A. (2014). Musically cued gait-training improves both perceptual and motor timing in Parkinson's disease. Frontiers in Human Neuroscience, 8, 494.

The invention has been made in the context of the implementation of the BAASTA assessment tool but it may prove useful in some other application as well.

A few tasks implemented in BAASTA deal with the synchronization of a reference beat sequence and a recorded one (i.e., the tapping sequence).

A reference beat sequence is herein defined as a sound sequence containing sound beats with a known pace used as a reference. The pace of the reference beat sequence is typically regular but not necessarily. For some task, for example the adaptive tapping task, the pace may vary at the end of the sequence. The sound beat sequence may take the form of a sequence of discrete sounds, such as a metronome. It may also take the form of a musical sequence or even a combination of the two. The resulting data used for the synchronization is the sequence of the times of occurrence of the beats, namely a sequence of time-codes based on a reference clock.

The tapping sequence is herein defined as the sequence of time-codes of some kind of tapping by the subject based on the same reference clock. The tapping itself may take numerous forms. The subject may be asked to tap with his hand or finger, to produce a sound, to produce a movement. This tapping is recorded in an adapted way. The record may be audio if the tapping results in a sound, it may be video if the tapping results in a recognizable movement, it may also be recorded by an electronic interface of a computing device like a touch-screen, a keyboard connected to a computer. Whatever the actual tap type and the actual tap recording method, the time of occurrence of each tap is extracted to produce the tapping sequence as a sequence of time-codes. These time-codes are expressed relatively to the same clock reference than the reference beat sequence.

The synchronization process is herein defined as the computation of statistics on the tapping sequence and its correspondence to the reference beat sequence. It aims at computing an estimate of how close the tapping sequence is in regard to the reference beat sequence. It typically comprises the computation of an estimate of the synchronization accuracy corresponding to a measure of the distance between the tap and the associated reference beat. It also comprises typically the computation of the synchronization variability corresponding to the variability of the previous estimate.

The synchronization process is implemented by a computing device appropriately programmed taking in input the reference beat sequence and the tapping sequence and producing a score corresponding to the rhythmic ability of a subject.

With a subject presenting good rhythmic ability, the association of taps with reference beats is straightforward as explained in the preamble. But when facing a subject with poor rhythmic ability this association is far more complicated. Reliable statistics depends on quite continuous and regular sequences of taps.

According to the invention, the synchronization process includes filtering steps prior to the synchronization itself in order to prevent inaccurate or unreliable measures of synchronization accuracy and variability.

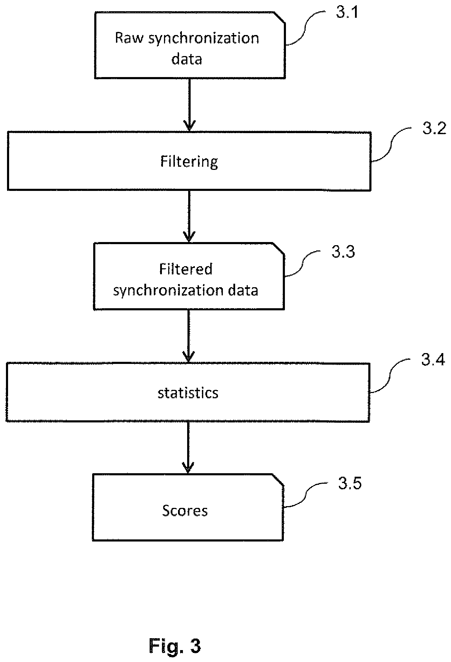

**Figure 3** illustrates the general process of synchronization according to an embodiment of the invention.

Data **3.1** represents the raw synchronization data, namely the reference beat sequence and the recorded tap sequence as defined previously.

These raw synchronization data are filtered by a filtering module in step **3.2.** The aim of this filtering step is to produce from the raw tapping sequence at least one sequence of filtered synchronization data **3.3** having some good properties in term of regularity and continuity. Details of the filtering will be described lately.

Some statistics are computed by the statistic module **3.4** in order to compute some scores **3.5.** These scores reflect some rhythmic skills of the subject. They typically comprise estimation of the synchronization accuracy and of the synchronization variability.

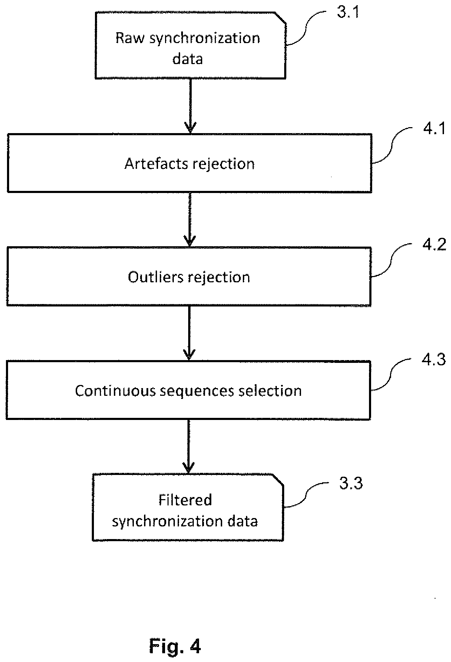

**Figure 4** illustrates the filtering process in an embodiment of the invention.

In a first step **4.1,** the raw data **3.1** are subjected to a first filter to reject artefacts. Artefacts are defined as a tap for which the inter-tap-interval between the actual tap and the previous one is smaller than a given threshold. The idea is to reject taps occurring very soon after another one. These artefacts may be created by the tap recognition process or by the dedicated device. In our preferred embodiment, the threshold is fixed to 150 milliseconds. This threshold may depend on the tap recognition process and adjusted based on the actually produced sequences.

In a second step **4.2,** a second filter takes place to reject outliers. Outliers are defined as a tap for which the inter-tap-interval between the actual tap and the previous one is outside a given range. The actual range is typically given by a low threshold and a high threshold. All taps with an inter-tap-interval smaller than the low threshold or greater than the high threshold are discarded. In our preferred embodiment, the low and high thresholds are computed based on statistics on the sequence of inter-tap-intervals computed on the entire tapping sequence. As any sequence of data this sequence of inter-tap-intervals may be statistically analyzed as a distribution of data to compute three well-known statistics values.

The first one is the first quartile (Q1) corresponding to the value of the inter-tap-interval of the 25th percentile. The second one is the third quartile (Q3) corresponding to the value of the inter-tap-interval of the 75th percentile. The third one is the interquartile range (IQR), also called the midspread or middle fifty, is a measure of statistical dispersion, being equal to the difference between the upper and lower quartiles. In other words, the interquartile range is the 1st quartile subtracted from the 3rd quartile (IQR=Q3-Q1).

In our preferred embodiment:

- $$Low\ threshold = Q1 - 3.IQR$$

- $$High\ threshold = Q3 + 3.IQR$$

where the "." means the multiplication.

In a third step **4.3**, a third filter is used to select continuous sequences of taps. A continuous sequence of taps is defined as a sequence of successive taps without any outliers and having a minimum length. In other words, a continuous sequence is a sequence of successive taps between two successive outliers. The minimum length is defined by a parameter given between 2 and the total length of the sequence. In our preferred embodiment the minimum length is fixed to 3.

These continuous sequences may be used for the computation of the scores using the statistic module. It is worth noting that the filtering is likely to result in a plurality of individual sequences. For the purpose of statistic computation, these individual sequences are treated as a single sequence being the concatenation of all individual sequences.

In the invention, a fourth step not represented is used to apply a fourth filter in order to select regular sequences among continuous sequences. A regular sequence is a sequence of tap with a strict one-to-one relationship with the reference beats. This is achieved by dividing the time into intervals centred on beats with a duration corresponding to the inter-beat-interval. These intervals correspond to the intervals marked by dotted lines on **figure 1** and **figure 2**. After this fourth filter, only sequences having at least 3 synchronized taps (regular and continuous taps) are kept for further analyses.

The statistic module to compute scores from the filtered synchronization data is presented in two main embodiments.

In a first embodiment of the statistic module, statistics are computed according to linear statistics. Scores are defined as comprising the synchronization accuracy and the synchronization variability. In this embodiment the synchronization accuracy (SA) is computed as a mean absolute asynchrony. The asynchrony of a tap is defined by the difference between the time of the tap and the time of the corresponding beat. Namely the synchronization accuracy is given by the following equation:

$$SA = \frac{\sum_{i=1}^{N} |tap_i - beat_i|}{N};$$

where $N$ is the total number of taps in the filtered synchronization data, $tap_i$ is the time of the $i^{th}$ tap in the filtered sequence and $beat_i$ the time of the corresponding beat. In this embodiment the synchronization variability (SV) is computed as the standard error of the asynchrony. Namely it is given by the equation:

$$SV = \frac{Standard\ Deviation(asynchrony)}{\sqrt{N}};$$

where the Standard Deviation is the common statistic function.

It is worth noting that linear statistics works particularly well on regular and continuous sequences. In absence of the fourth filter, namely with only continuous sequences the fact that for some taps the one-to-one relationship with the beat is not guaranteed may hinder the computation of asynchrony, thus leading to tap rejection. This fact degrades the accuracy of scores. In our preferred embodiment, the optional fourth filter to select regular sequences is always used in conjunction with linear statistics.

In an example of the statistic module, not being part of the invention, statistics are computed according to circular statistics. Scores are still defined as comprising the synchronization accuracy and the synchronization variability.

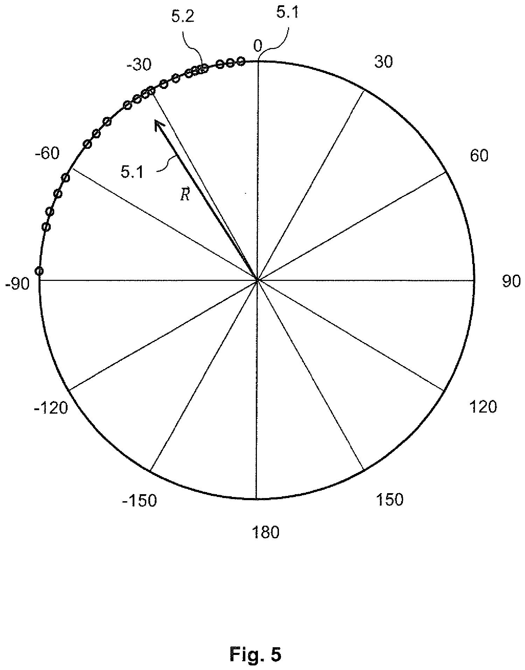

Circular statistics are computed on a circle as illustrated on **figure 5,** where data are represented on a polar scale. The circle represents the inter-beat-interval centred on the beat. Namely, the time of the beat corresponds to the 0 position **5.1.** Each tap time is converted using polar coordinates into a vector **5.2** on the unitary circle illustrated by a little circle on **figure 5.** The angle of the vector $\vec{r_i}$ corresponding to a tap is therefore computed as $angle(\vec{r_i}) = 2.\ PI.\ (tap_i/IBI),$ where $tap_i$ is

the time of the tap and *IBI* the inter-beat-interval.

Circular statistics provide a description of the distribution of taps' relative phase (with respect to the time of the beat stimulus) within the IBI which, by definition, does not require that the taps have a 1-to-1 relation with the beats. This approach is compatible with the possibility that there are missing taps or that there is more than one tap within the same beat interval. In this case synchronization accuracy and variability for a sequence of taps are not computed as averages of individual differences (or asynchronies) between one beat and one and only one tap event, but rather as properties of the entire distribution of relative phases with respect to the time of the beats.

It is worth noting that circular statistics works well on continuous sequences. Having regular sequences is neither mandatory nor especially advantageous here. In our preferred embodiment the fourth filter to select regular sequences is not used when statistics are computed using circular statistics.

The vector $\vec{R}$ is the resultant vector of all $\vec{r_i}$, namely it corresponds to the vector sum of the $\vec{r_i}$ divided by the number *N* of vectors:

$$\vec{R} = \frac{\sum_{i=1}^{n} \vec{r_i}}{N}.$$

The angle of $\vec{R}$ gives an estimate of the phase shift of the tap relative to the beats while the length of $\vec{R}$, namely $|\vec{R}|$, gives an estimate of the distribution of the $\vec{r_i}$. The synchronization accuracy SA and the synchronization variability SV are given by the following equations:

$$SA = angle(\vec{R});$$

$$SV = 1 - |\vec{R}|;$$

Alternatively to the synchronization variability SV, the synchronization consistency SC may be used. The synchronization consistency is given by the following equation:

$$SC = |\vec{R}|;$$

Advantageously, the data are submitted to the Raleigh's test to determine whether the distribution may be qualified as random or rather exhibits a particular phase shift relative to the beats sequence.

The Rayleigh test allows assessing how large the resultant vector length R must be to indicate a non-uniform distribution. It is particularly well-suited for detecting a unimodal deviation from uniformity. The approximate p-value is computed as:

$$P = exp\left[\sqrt{\left(1 + 4N + 4(N^2 - R_n^2)\right)} - (1 + 2N)\right]$$

where $R_n = R. N$; $R = |\vec{R}|$ and *N* is the number of samples. This approximation is valid up to three decimal places for *N* as small as 10. The Rayleigh test can also be applied to axial data after suitable transformation. Importantly, it assumes sampling from a von Mises distribution. A small p indicates a significant departure from uniformity and indicates to reject the hypothesis of a uniform distribution. The p criterion for statistical significance is set to .05, which is standard in experimental and behavioural sciences. Importantly, when the Rayleigh test is not statistically significant, the angle of $\vec{R}$ cannot be interpreted, thus no reliable measure of SA can be provided in this case.

Some detailed description on circular statistics may be found in "CircStat: A MATLAB Toolbox for Circular Statistics", Philipp Berens, Journal of Statistical Software, September 2009, Volume 31, Issue 10.

In an example not being part of the invention, where the beat sequence is constituted by a musical excerpt, the synchronization may occur at different metrical levels. Actually, a musical excerpt exhibits several metrical levels, for example corresponding to the duration of a measure, a half note, or a quarter note. The subject asked to produce a tapping sequence based on the musical excerpt may choose one of these levels to synchronize with. It is therefore important to detect the metrical level chosen by the subject to obtain accurate scores. In this particular example circular statistics are computed as described above and the resultant vector $\vec{R}$ is computed for each possible inter-beat-interval according to each possible metrical level in the music. The inter-beat-interval corresponding to the maximum length of $\vec{R}$ gives the chosen metrical level. The sequence of reference beat times is filtered to only take into account beats corresponding to the chosen metrical level. These computations constitute a further filtering step in the filtering module before applying either linear or circular statistics to produce scores.

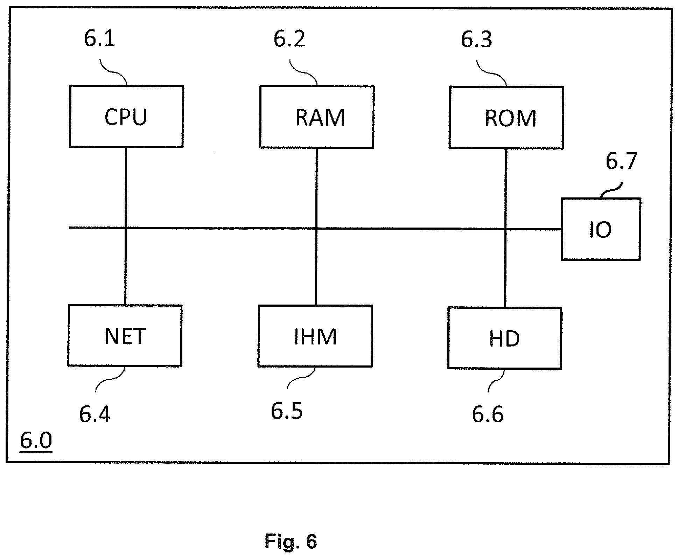

**Figure** 6 is a schematic block diagram of a computing device **6.0** for implementation of one or more embodiments of the invention. The computing device **6.0** may be a device such as a micro-computer, a workstation or a light portable device. The computing device **6.0** comprises a communication bus connected to:

- a central processing unit **6.1,** such as a microprocessor, denoted CPU;
- a random access memory **6.2,** denoted RAM, for storing the executable code of the method of embodiments of the invention as well as the registers adapted to record variables and parameters necessary for implementing the method for encoding or decoding at least part of an image according to embodiments of the invention, the memory capacity thereof can be expanded by an optional RAM connected to an expansion port for example;
- a read only memory **6.3,** denoted ROM, for storing computer programs for implementing embodiments of the invention;
- a network interface **6.4** is typically connected to a communication network over which digital data to be processed are transmitted or received. The network interface **6.4** can be a single network interface, or composed of a set of different network interfaces (for instance wired and wireless interfaces, or different kinds of wired or wireless interfaces). Data packets are written to the network interface for transmission or are read from the network interface for reception under the control of the software application running in the CPU **6.1;**
- a user interface **6.5** may be used for receiving inputs from a user or to display information to a user;
- a hard disk **6.6** denoted HD may be provided as a mass storage device;
- an I/O module **6.7** may be used for receiving/sending data from/to external devices such as a video source or display.

The executable code may be stored either in read only memory **6.3,** on the hard disk **6.6** or on a removable digital medium such as for example a disk. According to a variant, the executable code of the programs can be received by means of a communication network, via the network interface **6.4,** in order to be stored in one of the storage means of the communication device **6.0,** such as the hard disk **6.6,** before being executed.

The central processing unit **6.1** is adapted to control and direct the execution of the instructions or portions of software code of the program or programs according to embodiments of the invention, which instructions are stored in one of the aforementioned storage means. After powering on, the CPU **6.1** is capable of executing instructions from main RAM memory **6.2** relating to a software application after those instructions have been loaded from the program ROM **6.3** or the hard-disc (HD) **6.6** for example. Such a software application, when executed by the CPU **6.1,** causes the steps of the flowcharts shown in **Figures 3** to **4** to be performed.

Any step of the algorithm shown in **Figure 3** and **4** may be implemented in software by execution of a set of instructions or program by a programmable computing machine, such as a PC ("Personal Computer"), a DSP ("Digital Signal Processor") or a microcontroller; or else implemented in hardware by a machine or a dedicated component, such as an FPGA ("Field-Programmable Gate Array") or an ASIC ("Application-Specific Integrated Circuit").

The main and most relevant effect of these new filtering procedures is that they allow analyzing human synchronization data to an external sound stimuli even when data is relatively « noisy » such as is the case of patients with rhythm disorders and in developmental populations. Note that the domain of application of the technique is not confined to synchronization to sound stimuli and to the tapping paradigm. The method can be used generally to filter synchronization data between various classes of discrete human movement (arm, leg, hand and finger movement) synchronized to a variety of predictable external stimuli (sound, visual, tactile, somatosensory), with a simple rhythmic structure or with a complex rhythm, such as music. The described filtering methods provide reliable input data for the computation of synchronization accuracy and variability using both linear statistics and circular statistics. The advantage of linear statistics is that the measures of synchronization using this method are quite widespread and can reliably quantify rhythmic skills in particular in individuals with a given degree of rhythmic expertise (e.g., musicians). However, the use of linear statistics, because they work better with a one-to-one correspondence between motor events (taps) and the beats, is not appropriate to quantify rhythmic skills in individuals with rhythmic disorders. A good alternative is represented by circular statistics, which, after data filtering as described, allow researchers and clinicians to compute reliable measures of synchronization skills even in these populations.

An example of application in which the filtering methods described in this document are used is a recently developed battery for testing rhythmic skills based on auditory perceptual tasks and tapping tasks for healthy individuals and populations with rhythm disorders (e.g., with Parkinson's disease). The evaluation tool is the Battery for the Assessment of Auditory Sensorimotor and Timing Abilities (BAASTA). The BAASTA includes 10 tasks, 5 testing perceptual timing and 5 sensorimotor timing. The battery allows firstly to assess timing abilities in both beat-based and interval-timing tasks, and secondly to test both perception and sensorimotor timing using the same simple and complex auditory stimuli (i.e., a metronome vs. musical stimuli). The filtering methods indicated in this document are used to analyze finger tapping data in 4 of the sensorimotor timing tasks of the BAASTA (Paced tapping to a metronome, Paced tapping to music, Synchronization-continuation, Adaptive tapping task). The use of the aforementioned systematic procedures has allowed obtaining reliable measures of synchronization accuracy and variability (or consistency) for example in patients with Parkinson's disease, and children with developmental stuttering. These measures were capable of showing profiles of rhythmic impairments and of identifying individual differences in these populations in terms of rhythmic skills. This information provides highly valuable guidelines to create individualized rehabilitation strategies based on rhythmic training.

Although the present invention has been described hereinabove with reference to specific embodiments, the present invention is not limited to the specific embodiments, and modifications will be apparent to a skilled person in the art which lie within the scope of the present invention.

Many further modifications and variations will suggest themselves to those versed in the art upon making reference to the foregoing illustrative embodiments, which are given by way of example only and which are not intended to limit the scope of the invention, that being determined solely by the appended claims. In particular the different features from different embodiments may be interchanged, where appropriate.

In the claims, the word "comprising" does not exclude other elements or steps, and the indefinite article "a" or "an" does not exclude a plurality. The mere fact that different features are recited in mutually different dependent claims does not indicate that a combination of these features cannot be advantageously used.

**Claims**

1. A method for the synchronization of data sequences, the method comprising by a computing device:

    - obtaining a sequence of reference beat times;
    - obtaining a sequence of recorded tap times by a subject;
    - computing scores (3.5) reflecting some rhythmic skills of the subject, said scores comprising at least the synchronization accuracy and the synchronization variability of said sequence of recorded tap times regarding said sequence of reference beat times;
    said method further comprising a filtering step (3.2) prior to computing scores, the filtering step comprising:

        - rejecting artefacts in the sequence of recorded tap times (4.1), artefacts being defined as a tap for which the inter-tap-interval between the actual tap and the previous one is smaller than a given threshold;
        - rejecting outliers in the sequence of recorded tap times (4.2), outliers being defined as a tap for which the inter-tap-interval between the actual tap and the previous one is outside a given range; and
        - selecting continuous sequences of taps in the sequence of recorded tap times (4.3); a continuous sequence of taps being defined as a sequence of successive taps without any outliers and having a given minimum length; the step of computing scores (3.5) comprises computing the synchronization accuracy and the synchronization variability of said sequence of recorded tap times regarding said sequence of reference beat times according to linear statistics,

    **characterised in that** the filtering step (3.2) further comprises selecting regular sequences of taps in the sequence of recorded tap times among continuous sequences; a regular sequence being a sequence of taps with a strict one-to-one relationship with the reference beats.

2. The method of claim 1, wherein the given range is the range between a low threshold and a high threshold given by:

    - the low threshold is equal to Q1 - 3.IQR; and
    - the high threshold is Q3 + 3.IQR;
    wherein Q1 is the first quartile, Q3 is the third quartile and IQR is the interquartile range of the inter-tap-interval sequence.

3. The method according to any one claim 1 or 2, wherein the sequence of reference beat times corresponding to a musical excerpt with several metrical level, the filtering step (3.2) further comprises:

    - computing a resultant vector $\vec{R}$ according to circular statistics for each possible inter-beat-interval according to each possible metrical level in the music;
    - determining the metrical level chosen by the subject as the inter-beat-interval corresponding to the maximum length of $\vec{R}$; and
    - filtering the sequence of reference beat times to only take into account beats corresponding to the chosen metrical level.

4. A device for the synchronization of data sequences, the device comprising:

- means for obtaining a sequence of reference beat times;
- means for obtaining a sequence of recorded tap times by a subject;
- means for computing scores reflecting some rhythmic skills of the subject, said scores comprising at least the synchronization accuracy and the synchronization variability of said sequence of recorded tap times regarding said sequence of reference beat times;

the device further comprising a filtering module comprising:

- means for rejecting artefacts in the sequence of recorded tap times, artefacts being defined as a tap for which the inter-tap-interval between the actual tap and the previous one is smaller than a given threshold;
- means for rejecting outliers in the sequence of recorded tap times, outliers being defined as a tap for which the inter-tap-interval between the actual tap and the previous one is outside a given range; and
- means for selecting continuous sequences of taps in the sequence of recorded tap times; a continuous sequence of taps being defined as a sequence of successive taps without any outliers and having a given minimum length;

the means for computing scores is configured to compute the synchronization accuracy and the synchronization variability of said sequence of recorded tap times regarding said sequence of reference beat times according to linear statistics,
**characterised in that** the filtering module further comprises means for selecting regular sequences of taps in the sequence of recorded tap times among continuous sequences; a regular sequence being a sequence of taps with a strict one-to-one relationship with the reference beats.

5. A computer program product for a programmable apparatus, the computer program product comprising a sequence of instructions for implementing a method according to any one of claims 1 to 3, when loaded into and executed by the programmable apparatus.

6. A computer-readable storage medium storing instructions of a computer program for implementing a method according to any one of claims 1 to 3.

**Patentansprüche**

1. . Verfahren zur Synchronisierung von Datensequenzen, wobei das Verfahren durch eine Rechenvorrichtung umfasst:

- Erhalten einer Sequenz von Referenzschlagzeiten;
- Erhalten einer Sequenz aufgezeichneter Tippzeiten durch ein Subjekt;
- Berechnen von Bewertungen (3.5), die bestimmte rhythmische Fähigkeiten des Subjekts wiedergeben, wobei die Bewertungen mindestens die Synchronisierungsgenauigkeit und die Synchronisierungsvariabilität der Sequenz aufgezeichneter Tippzeiten bezüglich der Sequenz von Referenzschlagzeiten umfassen;

wobei das Verfahren weiter einen Filterschritt (3.2) vor dem Berechnen der Bewertungen umfasst, wobei der Filterschritt umfasst:

- Zurückweisen von Artefakten in der Sequenz aufgezeichneter Tippzeiten (4.1), wobei Artefakte als ein Tipp definiert werden, bei dem das Zwischentippintervall zwischen dem aktuellen Tipp und dem vorherigen kleiner ist als ein gegebener Schwellenwert;
- Zurückweisen von Ausreißern in der Sequenz aufgezeichneter Tippzeiten (4.2), wobei Ausreißer als ein Tipp definiert werden, bei dem das Zwischentippintervall zwischen dem aktuellen Tipp und dem vorherigen außerhalb eines gegebenen Bereichs liegt; und
- Auswählen kontinuierlicher Sequenzen von Tipps in der Sequenz aufgezeichneter Tippzeiten (4.3); wobei eine kontinuierliche Sequenz von Tipps als eine Sequenz aufeinanderfolgender Tipps ohne Ausreißer und eine bestimmte Mindestlänge aufweisend definiert wird;
der Schritt des Berechnens von Bewertungen (3.5) das Berechnen der Synchronisierungsgenauigkeit und der Synchronisierungsvariabilität der Sequenz aufgezeichneter Tipps bezüglich der Sequenz von Referenzschlagzeiten gemäß linearen Statistiken umfasst, **dadurch gekennzeichnet, dass** der Filterschritt (3.2) weiter das Auswählen regelmäßiger Tippsequenzen in der Sequenz aufgezeichneter Tippzeiten unter kontinuierlichen Sequenzen umfasst; wobei eine regelmäßige Sequenz eine Tippsequenz mit einer strengen Eins-zu-Eins-Beziehung zu den Referenzschlägen ist.

2. Verfahren nach Anspruch 1, wobei der gegebene Bereich der Bereich zwischen einem unteren Schwellenwert und einem oberen Schwellenwert ist, der durch Folgendes gegeben ist:

- der untere Schwellenwert ist gleich Q1 - 3.IQR; und
- der obere Schwellenwert ist Q3 + 3. IQR;

wobei Q1 das erste Quartil, Q3 das dritte Quartil und IQR der Zwischenquartilbereich der Zwischentippintervall-Sequenz ist.

3. . Verfahren nach einem der Ansprüche 1 oder 2, wobei die Sequenz von Referenzschlagzeiten einem musikalischen Auszug mit mehreren metrischen Ebenen entspricht, wobei der Filterschritt (3.2) weiter Folgendes umfasst:

- Berechnen eines resultierenden Vektors $\vec{R}$ gemäß einer Kreisstatistik für jedes mögliche Zwischentippintervall gemäß jeder möglichen metrischen Ebene in der Musik;
- Bestimmen der von dem Subjekt ausgewählten metrischen Ebene als das Zwischentippintervall zwischen den Schlägen, das der maximalen Länge von $\vec{R}$ entspricht; und
- Filtern der Sequenz von Referenzschlagzeiten, um nur Schläge zu berücksichtigen, die der ausgewählten metrischen Ebene entsprechen.

4. . Vorrichtung zur Synchronisierung von Datensequenzen, wobei die Vorrichtung umfasst:

- Mittel zum Erhalten einer Sequenz von Referenzschlagzeiten;
- Mittel zum Erhalten einer Sequenz durch ein Subjekt aufgezeichneter Tippzeiten;
- Mittel zum Berechnen von Bewertungen, die bestimmte rhythmische Fähigkeiten des Subjekts wiedergeben, wobei die Bewertungen mindestens die Synchronisierungsgenauigkeit und die Synchronisierungsvariabilität der Sequenz aufgezeichneter Tippzeiten bezüglich der Sequenz von Referenzschlagzeiten umfassen;

wobei die Vorrichtung weiter ein Filtermodul umfasst, umfassend:

- Mittel zum Zurückweisen von Artefakten in der Sequenz aufgezeichneter Tippzeiten, wobei Artefakte als ein Tipp definiert werden, bei dem das Zwischentippintervall zwischen dem aktuellen Tipp und dem vorherigen kleiner ist als ein gegebener Schwellenwert;
- Mittel zum Zurückweisen von Ausreißern in der Sequenz aufgezeichneter Tippzeiten, wobei Ausreißer als ein Tipp definiert werden, bei dem das Zwischentippintervall zwischen dem aktuellen Tipp und dem vorherigen außerhalb eines gegebenen Bereichs liegt; und
- Mittel zum Auswählen kontinuierlicher Sequenzen von Tipps in der Sequenz aufgezeichneter Tippzeiten; wobei eine kontinuierliche Sequenz von Tipps ist als eine Sequenz aufeinanderfolgender Tipps ohne Ausreißer und eine bestimmte Mindestlänge aufweisend definiert wird;
die Mittel zum Berechnen von Bewertungen dazu konfiguriert sind, die Synchronisierungsgenauigkeit und die Synchronisierungsvariabilität der Sequenz aufgezeichneter Tipps bezüglich der Sequenz von Referenzschlagzeiten gemäß linearer Statistiken zu berechnen, **dadurch gekennzeichnet, dass** das Filtermodul weiter Mittel zum Auswählen regelmäßiger Tippsequenzen in der Sequenz aufgezeichneter Tippzeiten unter kontinuierlichen Sequenzen umfasst; wobei eine regelmäßige Sequenz eine Tippsequenz mit einer strengen Eins-zu-Eins-Beziehung zu den Referenzschlägen ist.

5. . Computerprogrammprodukt für eine programmierbare Einrichtung, wobei das Computerprogrammprodukt eine Sequenz von Anweisungen zum Implementieren eines Verfahrens nach einem der Ansprüche 1 bis 3 umfasst, wenn es in die programmierbare Einrichtung geladen und von dieser ausgeführt wird.

6. . Computerlesbares Speichermedium, das Anweisungen eines Computerprogramms zum Implementieren eines Verfahrens nach einem der Ansprüche 1 bis 3 speichert.

**Revendications**

1. Procédé de synchronisation de séquences de données, le procédé comprenant par l'intermédiaire d'un dispositif informatique les étapes consistant à :

   - obtenir une séquence de temps de battement de référence ;
   - obtenir une séquence de temps de tapotement enregistrés par un sujet ;
   - calculer des scores (3.5) reflétant certaines aptitudes rythmiques du sujet, lesdits scores comprenant au moins la précision de synchronisation et la variabilité de synchronisation de ladite séquence de temps de tapotement enregistrés par rapport à ladite séquence de temps de battement de référence ;
   ledit procédé comprenant en outre une étape de filtrage (3.2) avant le calcul des scores, l'étape de filtrage comprenant les étapes consistant à :

   - rejeter les artefacts dans la séquence des temps de tapotement enregistrés (4.1), les artefacts étant définis comme un tapotement pour lequel l'intervalle inter-tapotements entre le tapotement actuel et le précédent est inférieur à un seuil donné ;
   - rejeter des valeurs aberrantes dans la séquence des temps de tapotement enregistrés (4.2), les valeurs aberrantes étant définies comme un tapotement pour lequel l'intervalle inter-tapotements entre le tapotement actuel et le tapotement précédent se situe en dehors d'un intervalle donné ; et
   - sélectionner des séquences continues de tapotements dans la séquence des temps de tapotement enregistrés (4.3) ; une séquence continue de tapotements étant définie comme une séquence de tapotements successifs sans aucune valeur aberrante et ayant une longueur minimale donnée ;

   l'étape de calcul des scores (3.5) comprend le calcul de la précision de synchronisation et de la variabilité de synchronisation de ladite séquence de temps de tapotements enregistrés par rapport à ladite séquence de temps de battement de référence selon des statistiques linéaires,
   **caractérisé en ce que** l'étape de filtrage (3.2) comprend en outre une étape consistant à sélectionner, parmi des séquences continues, des séquences régulières de tapotement dans la séquence des temps de tapotement enregistrés ; une séquence régulière étant une séquence de tapotements ayant une relation stricte de un à un avec les battements de référence.

2. Procédé de la revendication 1 dans lequel l'intervalle donné est l'intervalle entre un seuil bas et un seuil haut donné par :

   - le seuil bas est égal à QI - 3.IQR ; et
   - le seuil haut est Q3 + 3.IQR

   dans lequel Q1 est le premier quartile, Q3 est le troisième quartile et IQR est l'intervalle interquartile de la séquence d'intervalle inter-tapotements.

3. Procédé selon l'une quelconque des revendications 1 ou 2, dans lequel la séquence de temps de battement de référence correspond à un extrait musical avec plusieurs niveaux métriques, l'étape de filtrage (3.2) comprend en outre les étapes consistant à :

   - calculer un vecteur résultant $\vec{R}$ en fonction de statistiques circulaires pour chaque intervalle inter-battement possible en fonction de chaque niveau métrique possible dans la musique ;
   - déterminer le niveau métrique choisi par le sujet comme l'intervalle inter-battement correspondant à la longueur maximale de $\vec{R}$ ; et
   - filtrer la séquence des battements de référence pour ne prendre en compte que les battements correspondant au niveau métrique choisi.

4. Dispositif pour la synchronisation de séquences de données, le dispositif comprenant :

   - des moyens pour obtenir une séquence de temps de battement de référence ;
   - des moyens pour obtenir une séquence de temps de tapotement enregistrés par un sujet ;
   - des moyens de calcul des scores reflétant certaines aptitudes rythmiques du sujet, lesdits scores comprenant au moins la précision de synchronisation et la variabilité de synchronisation de ladite séquence de temps de tapotement enregistrés par rapport à ladite séquence de temps de battement de référence ;

le dispositif comprend en outre un module de filtrage comprenant :

- des moyens pour rejeter des artefacts dans la séquence des temps de tapotement enregistrés, les artefacts étant définis comme un tapotement pour lequel l'intervalle inter-tapotement entre le tapotement actuel et le précédent est inférieur à un seuil donné ;
- des moyens pour rejeter des valeurs aberrantes dans la séquence des temps de tapotement enregistrés, les valeurs aberrantes étant définies comme un tapotement pour lequel l'intervalle inter-tapotements entre le tapotement actuel et le précédent est en dehors d'un intervalle donné ; et
- des moyens pour sélectionner des séquences continues de tapotements dans la séquence des temps de tapotement enregistrés ; une séquence continue de tapotements est définie comme une séquence de tapotements successifs sans aucune valeur aberrante et ayant une longueur minimale ;

les moyens de calcul des scores sont configurés pour calculer la précision de synchronisation et la variabilité de synchronisation de ladite séquence de temps de tapotement enregistrés par rapport à ladite séquence de temps de battement de référence selon des statistiques linéaires,
**caractérisé en ce que** le module de filtrage comprend en outre des moyens de sélection de séquences régulières de tapotements dans la séquence des temps de tapotement enregistrés parmi des séquences continues ; une séquence régulière est une séquence de tapotements ayant une relation stricte de un à un avec les battements de référence.

5. Produit de programme d'ordinateur pour un appareil programmable, le produit de programme d'ordinateur comprenant une séquence d'instructions pour la mise en œuvre d'un procédé selon l'une quelconque des revendications 1 à 3, lorsqu'il est chargé dans l'appareil programmable et exécuté par celui-ci.

6. Support de stockage lisible par ordinateur stockant des instructions d'un programme d'ordinateur pour la mise en œuvre d'un procédé selon l'une quelconque des revendications 1 à 3.

**Fig. 1**

**Fig. 2**

```
                    ┌─────────────────────┐
                    │ Raw synchronization │──── 3.1
                    │       data          │
                    └──────────┬──────────┘
                               │
                               ▼
        ┌──────────────────────────────────────────────┐
        │                Filtering                      │──── 3.2
        └──────────────────────┬───────────────────────┘
                               │
                               ▼
                    ┌─────────────────────┐
                    │      Filtered       │──── 3.3
                    │ synchronization data│
                    └──────────┬──────────┘
                               │
                               ▼
        ┌──────────────────────────────────────────────┐
        │                statistics                     │──── 3.4
        └──────────────────────┬───────────────────────┘
                               │
                               ▼
                    ┌─────────────────────┐
                    │       Scores        │──── 3.5
                    └─────────────────────┘
```

**Fig. 3**

Raw synchronization
data — 3.1

Artefacts rejection — 4.1

Outliers rejection — 4.2

Continuous sequences selection — 4.3

Filtered
synchronization data — 3.3

**Fig. 4**

**Fig. 5**

Fig. 6

**EP 3 115 916 B1**

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **BROCHARD et al.** Evidence of beat perception via purely tactile stimulation. *Brain Research*, 2008, vol. 1223, 59-64
- **BENOIT, C-E.** ; **DALLA BELLA, S.** ; **FARRUGIA, N.** ; **OBRIG, H.** ; **MAINKA, S** ; **KOTZ, S.A.** Musically cued gait-training improves both perceptual and motor timing in Parkinson's disease. *Frontiers in Human Neuroscience*, 2014, vol. 8, 494
- **PHILIPP BERENS**. CircStat: A MATLAB Toolbox for Circular Statistics. *Journal of Statistical Software*, September 2009, vol. 31 (10)